(19) Europäisches Patentamt
European Patent Office
Office européen des brevets

(11) **EP 1 921 447 A1**

(12) **EUROPEAN PATENT APPLICATION**

(43) Date of publication:
**14.05.2008 Bulletin 2008/20**

(51) Int Cl.:
***G01N 33/12*** *(2006.01)*  ***G01N 27/403*** *(2006.01)*

(21) Application number: **06123613.9**

(22) Date of filing: **07.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicants:
- **Østerlie, Marianne**
  **7052 Trondheim (NO)**
- **Hannisdal, Atle**
  **7030 Trondheim (NO)**

(72) Inventors:
- **Østerlie, Marianne**
  **7052 Trondheim (NO)**

- **Hannisdal, Atle**
  **7030 Trondheim (NO)**

(74) Representative: **Plougmann & Vingtoft A/S**
**Sundkrogsgade 9**
**P.O. Box 831**
**2100 Copenhagen Ø (DK)**

Remarks:
•The application is published incomplete as filed (Article 93 (2) EPC).
•A request for correction of the claims numbering has been filed pursuant to Rule 88 EPC. A decision on the request will be taken during the proceedings before the Examining Division (Guidelines for Examination in the EPO, A-V, 3.).

(54) **Method and device for determining hypoxanthine in biological samples**

(57) A process for determining a hypoxanthine content in a muscle sample using a direct voltammetric measurement of hypoxanthine concentration ($C_{Hx}$) in a supernatant prepared from the muscle sample. The hypoxanthine content determined by voltammetry can be correlated with a freshness measure of a meat product from which the sample originates, e.g. QIM score evaluated by a sensory panel or K-value calculated from HPLC determination of ATP metabolites. A kit for preparing samples and a device for voltammetric determining $C_{Hx}$ and correlating $C_{HX}$ with freshness measure are disclosed, as well as a process for building data sets of voltammetric determined $C_{Hx}$ from meat products with known freshness measure for use in the correlation.

EP 1 921 447 A1

Fig. 4

**Description**

**Field of Invention**

The invention relates to a method for determining the freshness of raw, frozen or processed perishable fish, poultry and meat. More particularly, the invention relates to a method and a device for determining the level of hypoxanthine in a sample prepared from a biological tissue sample and correlating the hypoxanthine content to a freshness scale.

**Background of Invention**

Fish, poultry and meat freshness is fundamental to determine quality and can be described by a lot of properties. Since fish is generally more perishable than poultry and meat, the characterisation of freshness is more advanced for fish, and will receive the main focus in the following, without loss of generalisation.

Quality assurance in the fish sector involves monitoring defined quality criteria, properties assessed by a sensory method documented by an EU-scheme, as required by regulation (European Community, 1996). These properties are varying from one fish species to another and are dependent on biochemical changes occurring post mortem in fish (Huss, H, H. (1995). Quality and quality changes in fresh fish. FAO Fisheries Technical Paper 348, Rome, FAO). Unfortunately the scheme does not take differences between fish species into account. Another sensory method, the Quality Index method (QIM) using schemes developed for cod, herring as well as for redfish, sardines and flounder is used in the Nordic countries (Jonsdottir, S. (1992). Quality index method and TQM system. In Quality Issues in the Fish Industry (Olafsson, R and Ingthorsson, A.H. eds.). The Research Liaison Office, University of Iceland). The technique is based on selecting a number of quality attributes characteristic for a particular species and allocating scores to each attribute depending on the state of freshness or quality of the selected food item.

Fish industry is still reluctant to implement methods other than sensory to monitor freshness and quality of fish. The development of reliable methods to assess freshness of fish and other marine products has been a goal of fish research for many years. Several subjective and objective methods have been developed for this purpose. A review of some of the current methods is listed in Table 1. No one of these methods is used systematically by the industry today.

Table 1. Current methods for determining fish freshness.

| Method | Description |
| --- | --- |
| **Sensory evaluation** | |
| EU-scheme | Depends on sight, smell, taste, touch as judged by experienced persons |
| Quality Index Method (QIM) Chemical methods; | Used in the Nordic countries |
| Total volatile basic nitrogen | Measurement of trimethylamin, ammonia; volatile basic nitrogen by titration |
| Trimethylamin | Useful only for marine fish |
| Ammonia | Indicates only advanced spoilage |
| Volatile acids | Good correlation with bacterial spoilage |
| Nucleotide catabolism | Degradation products of ATP. Reliable quality indices of several fish/shellfish, based on K-value, indices of several fish/shellfish, based on K-value. Histamine is thermo stable and hence cooked fish can be |
| Histamine | analyzed by fluorimetric or HPLC methods |
| $H_2S$, $CH_3SH$, $(CH_3)_2S$ | Determination by gas chromatography |
| Rancidity | 2-Thiobarbituric acid (TBA) is a good index of oxidative rancidity. Reasonable correlation with sensory properties. Indicates more or less advanced spoilage |
| **Instrumental methods** | |
| Texture RT-freshmeter, Torrymeter | Intrinsic variations due to feeding seasons and so on Measuring electrical properties. Erroneous result caused by mechanical damage. Non-destructive. |
| Colour | Measuring colour of fillets. Non-destructive. On-line |
| Image analysis | Whole fish and fillets. Size, shape, visual defects |

(continued)

| Method | Description |
| --- | --- |
| **Sensory evaluation** | |
| VIS spectroscopy | NIR spectroscopy of fat, water and protein. Good correlation with sensory evaluation |
| Electronic nose | Electrochemical gas sensors monitoring CO, $H_2S$, NO, $NO_2$, and $SO_2$ |
| **Microbiological methods** | |
| Total plate count | Conventional techniques. Take 48-72 h. |
| Coli Fast | Rapid quantification of sulphide-producing bacteria. Take 1-24 h. |

In addition to Table 1 some conclusions from the Final Meeting of the Concerted Action "Evaluation of Fish Freshness in Nantes (1997) is given below (Methods to determine the freshness of fish in research and industry. Proceedings of the final meeting of the concerted action "evaluation of fish freshness" AIR3CT94 2283, Paris: Institute International du froid, 396 p):

Physical measurement give information on parameters related to fish freshness. None of these methods however, give a unique and unambiguous answer to whether the fish is fresh or not. Further characterisation of fish odours is needed before rapid assessment of volatile compounds in fish using gas sensors or electronic nose can be used as fish freshness detectors in the industry. Rapid methods in microbiology are in many cases not very rapid.

The conclusion is that sensory evaluation is the most important method in the industry to day. It has to be performed scientifically under carefully controlled conditions and by skilled and trained staff. The use of ATP metabolites as freshness indices is a research technique not widely used in the industry, but the K-value is a good freshness indicator. Reliable High Performance Liquid Chromatography (HPLC) methods are available but rapid, validated methods for use in the production are missing.

The Coli Fast method, taking 24 hours, has been tried out by the inventors and it is, in our opinion, far from being usable to evaluate fish freshness.

ATP metabolites

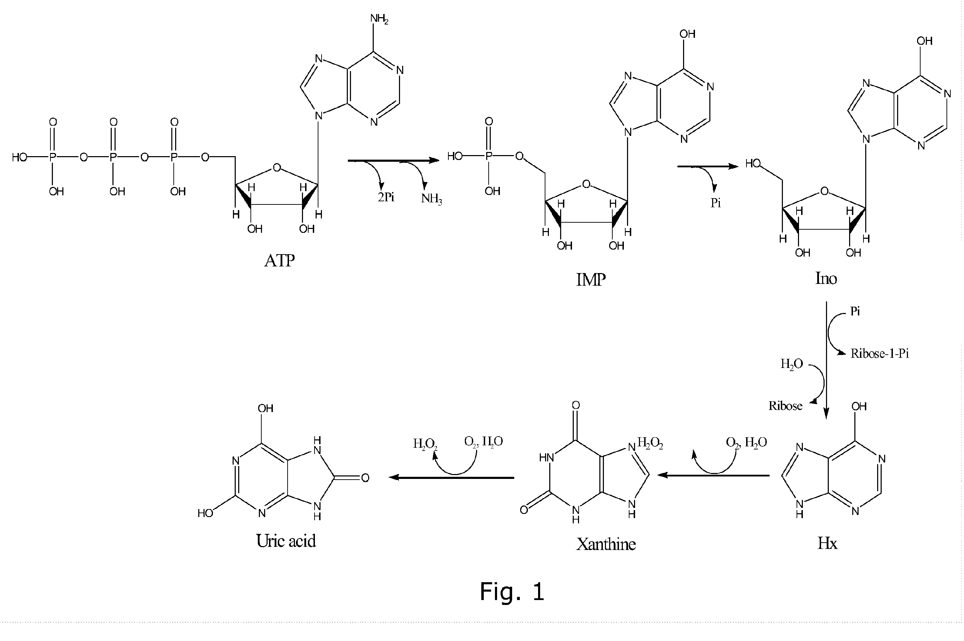

Adenosine triphosphate (ATP) is the primary source of energy in living organisms and the nucleotides produced from ATP are considered reliable and useful indicators of muscle quality. There is a strong correlation between nucleotide catabolism and loss of freshness in fish (Huss, 1995, Ashie et al. 1996). After death, ATP quickly decomposes to hypoxanthine, which further decomposes to xanthine and uric acid Because of the rate limiting step between xanthine and uric acid, inosine and hypoxanthine will accumulate in the muscle. The degradation process of ATP in muscle after death is shown in Figure 1.

Saito et al. (A new method for estimating the freshness in fish, Bull. Jpn. Soc. Sci. Fish. 24 (1959), 749-753) was the first to observe this degradation pattern and to develop a formula for fish freshness based on these autolytical changes, the K-value:

$$K(\%) = \frac{(Ino + Hx) * 100}{ATP + ADP + AMP + IMP + Ino + Hx} \qquad (1)$$

The compounds ATP, ADP, AMP, IMP, Ino, and Hx shown in the equation (1) represent the following:

ATP = Adenosine triphosphate
ADP = Adenosine diphosphate
AMP = Adenosine monophosphate
IMP = Inosine monophosphate
Ino = Inosine
Hx = Hypoxanthine

The K-value gives a relative freshness rating based primarily on the autolytic changes which take place during post mortem storage of the muscle. It is now widely accepted that IMP is responsible for the desirable fresh fish flavour and Hypoxanthine is considered to have a direct effect on the perceived bitter off-flavour of spoiled fish.

As mentioned in the above, HPLC methods for determining ATP metabolites and thereby a K-value are available. However, these methods a both time consuming and need a well trained staff. The equipment consisting of a chromatograph with a special column, pumps and an UV/VIS-detector is rather expensive and so are the six nucleotide standards requested. The HPLC instrument is unsuitable for a rapid measuring of food freshness in the industry or retail.

A number of prior art references using ATP metabolites such as hypoxanthine for determining freshness in fish, poultry and meat are presented in the following:

US 5,024,816 describes an apparatus for determining the degree of freshness of raw, frozen and processed fish, poultry and meat. The apparatus determines the amount of certain decomposition products of adenosine triphosphate such as hypoxanthine, inosine and inosinic acid by measuring the consumption of dissolved oxygen while each compound is subjected to action by certain enzymes.

US 4,105,800 relates to an immobilized enzyme method to assess fish quality.

US 5,288,613 discloses an enzyme-based biosensor system for monitoring the freshness of fish. The method comprises simultaneously determining, by use of a suitable amperometric electrode, the amount of 1) uric acid and hydrogen peroxide resulting from the degradation of hypoxanthine by xanthine oxidase, 2) the degradation of inosine by the combined action of nucleoside phosphorylase and xanthine oxidase, and 3) the degradation of inosine monophosphate by the combined action of nucleotidase, nucleoside phosphorylase and xanthine oxidase.

EP 0 137 677 describes a method for determining index of freshness of fish and molluscs by measuring the K-value of fish and molluscs.

DE 39 09 530 relates to determining xanthine or hypoxanthine content from sulphite and xanthine oxidase.

JP 62288561 discloses measurement of hypoxanthine and inosine by using not only an immobilized enzyme membrane excellent in reaction selectivity and a material exclusion property, but also a hydrogen peroxide electrode method.

JP 63015151 describes a method for quantifying uric acid and hypoxanthine in the same reaction tank continuously using an enzyme electrode for detecting uric acid.

Common disadvantages for these methods for determining decomposition products of ATP are that they are very time consuming and expensive in that they require laborious laboratory work based on enzyme reactions The use of enzymes has some disadvantages and limitations. Each type of enzyme has a pH- and temperature-optimum and the enzyme concentration has to fit the concentration of the target molecule. This is a disadvantage since the target molecule concentration is unknown when analysing various biological tissues. Purchasing enzymes is very expensive and can result in enzymes with different activities (IU) and storage life.

Hence, there is a need for a simple, fast and inexpensive method using ATP metabolites for determining freshness in fish, poultry and meat.

**Summary of the invention**

Accordingly, the invention preferably seeks to mitigate, alleviate or eliminate one or more of the above mentioned disadvantages singly or in any combination. In particular, it is an object of the present invention to solve the above mentioned problems of the prior art, by providing a simple, fast and inexpensive method using ATP metabolites for determining freshness in fish, poultry and meat.

In a first aspect, the invention provides a process for determining hypoxanthine content in a muscle sample comprising:

- adding at least one acid to a provided muscle sample to form a mixture having a pH<2, and homogenizing the resulting mixture to at least partly dissolve the sample;
- adding a base to the homogenized mixture to precipitate proteins and produce a supernatant comprising ATP metabolites from the muscle sample;
- diluting the supernatant with an buffer; and

- subjecting the diluted supernatant to adsorptive stripping voltammetry to determine a hypoxanthine concentration ($C_{Hx}$) in the diluted supernatant in comparison with a standard hypoxanthine solution.

Voltammetry is a well known electrochemical method often used for quantitative determination of organic and inorganic compounds in aqueous and non-aqueous solutions. The electrochemical activity of hypoxanthine is normally found in the heterocyclic ring (base). The electrochemical oxidation and reduction of hypoxanthine have been investigated by other scientists. A detailed electrochemical study of hypoxanthine helping to understand the oxidation mechanism and adsorption of purine bases and purine nucleosides is given in the article by Oliveira-Brett et al., Voltammetric and impedance studies of inosine-5'-monophosphate and hypoxanthine, Bioelectrochemistry 59 (2003), 49-56. They use a glassy carbon electrode to study Hypoxanthine oxidation. The article Ibrahim et al., Adsorptive stripping voltammetric behaviour of hypoxanthine, Analytica Chimica Acta 328 (1996), 47-52, describes the study of Hypoxanthine by adsorptive stripping voltammetry at a hanging mercury drop electrode. These references present studies on standard solutions; the methodology has never been tested on real biological samples extracted from fish, poultry and meat. Only one study on real samples has been found, (Jyn-Myng Zen et al., Electrocatalytic oxidation of hypoxanthine on a nafion/Lead-Ruthenium oxide pyrochlore modified electrode, Electroanalysis 12 (2000), 280-286). This article describes a chemically oxidation of hypoxanthine on a nafion/Lead-Ruthenium oxide pyrochlore modified electrode. In this study they use a chemically complex electrode (nafion/Lead-Ruthenium oxide pyrochlore modified electrode), which have to be regenerated before each analysis. The regeneration protocol is not very suitable for routine analysis.

In general, voltammetry provides very little or no information on the identity of the analytes. Due to the high quantitative sensitivity, varying electrochemical conditions in the sample solution may have a large impact on the result, and it is usually recommended that every sample should be prepared to minimize contamination of the sample solution applied in the voltammetric measurement. For this reason, laborious separation such as by liquid chromatography is typically used to separate individual analytes before analysis of degenerate samples. This adds to the complexity, time consumption and equipment requirements for using voltammetric methods.

ATP metabolites are the intermediates and products of ATP decomposition, occurring also in the enzymatic degradation of ATP after the death of an organism.

When analysing substances extracted from muscle samples, it is customary to chemically separate the substances, typically by some chromatographic technique, in order to be able to study the substances under pure or isolated conditions. In contradiction to this, the present invention applies biological solutions prepared directly from muscle samples. Thus, in the present context, the term "biological sample" is used to designate the solution used in the voltammetry, i.e. the diluted supernatant prepared from a muscle sample and not from a solution of purified or separated hypoxanthine. In contradiction to this, the term "pure samples" are used to designate hypoxanthine solutions of well-known composition, such as standard solutions, prepared from purified or separated hypoxanthine, typically containing no other substances potentially interfering with the voltammetric measurements of the analyte. Throughout the text, hypoxanthine is also denominated Hx and its concentration as $C_{Hx}$.

The present invention applies voltammetry to determine hypoxanthine content in biological samples prepared from muscle extracts. It is known that hypoxanthine can be studied by voltammetric techniques in solutions prepared from solutions of chemically pure hypoxanthine; see e.g. Oliveira-Brett et al. or Ibrahim et al., both referred in the above. The application of voltammetry on samples extracted directly from muscle tissue, and thereby containing miscellaneous biological active compounds, such as Hx and other ATP metabolites, in accordance with the present invention yields surprisingly good results. That this is not an obvious application of voltammetry is confirmed by that the prior art methods for determining Hx content in biological samples does not determine hypoxanthine content directly, but applies enzymatic reactions to degrade Hx to products detectable by electrochemical techniques, see e.g. US 5,288,613. Other indirect methods determine Hx degradation product by voltammetry, followed by calculation of $C_{Hx}$ using the equation of reaction. For the sake of clarity, it is stressed that voltammetric determined hypoxanthine content is a direct determination based on readings of the recorded voltammograms in comparison with voltammograms recorded on standard Hx solutions.

It is to be expected that the presence of other ATP metabolites in the voltammetric cell would disturb the Hx measurement by giving overlapping signals. However, it has turned out that this is only the case for Xanthine, the degradation product of Hx in Figure 1. Since the degradation of Hx is very slow, only insignificant amounts of Xanthine can be determined in meat from fish even after 8-10 days of storage. Only after 12-15 days does the Xanthine content significantly disturb the Hx determination, but at this time the meat is so degraded that determination of Hx content is less relevant.

Also, it is to be expected that redox active substances remnant from the dissolving of the meat sample would influence the working electrode to give an incorrect determination.

The biological samples applied in the present invention contain many different substances/analytes, including other ATP metabolites, water soluble peptides, amino acids and vitamins. As well as week salt solutions from the cytoplasm and NPN - (non protein nitrogen) compounds. Despite this fact and the technical prejudice that quantitative voltammetric determinations are best performed on pure Hx solutions or Hx degradation products, the inventors have contemplated direct voltammetric determination of Hx in biological samples prepared directly from muscle tissue. It is surprising that the Hx content can be relatively accurately determined in a biological sample containing several other potential analytes such as those listed in the above.

Preferably no further separation of the ATP metabolites is performed prior to the voltammetry. Hence, no further separation technique other that the precipitation caused by the addition of the base need to be carried out prior to the voltammetric measurements. This provides the advantage that expensive (in terms of equipment) and time-consuming separation techniques such as liquid chromatography are avoided.

The preferable chemical conditions in the supernatant and the intermediates in the sample preparation, e.g. pH, analyte concentration etc., will be described later. Also, some preferred acids, bases and diluents and their preferred concentrations and amounts corresponding to the preferred conditions will be described.

The voltammetric measurements applied in the process according to the present invention is a sensitive, easy-to-do electrochemical method. As will be described later, the outputs from the voltammetric measurements may be compared with sensory scores of QIM and an HPLC-method for the K-value for samples extracted from the same foodstuff product, stored under controlled conditions, e.g. on ice for fish, with good correlation. Thus, a data set of different $C_{Hx}$ determined by the process of the invention and corresponding sensory scores of QIM and/or K-values as determined by HPLC for the same foodstuff products may be build. Such data set may be correlated with $C_{Hx}$'s determined by the process of the invention for a foodstuff product of unknown freshness to estimate a QIM score and/or a K-value, and thereby days of storage, for the foodstuff product. This provides a fast, inexpensive, practical and objective way of assessing freshness of foodstuff products containing perishable fish, poultry or meat.

In the present description, a freshness measure may be any known of future quantitative measure of the age, eating quality, spoilage such as sensory and/or non-sensory measures comprising K-value, QIM, estimated number of days since slaughter, estimated number of days on storage, estimated remaining shelf-life, etc. Also, the foodstuff products may be any perishable product based on fish, poultry or meat, such as whole animals, cleaned fish, drawn poultry, butchered mammal, carcasses, filets, cuts, and other un-conserved fish, poultry or meat products.

Hence, it is preferred that:

- The process further comprises the step of correlating the determined $C_{Hx}$ to a freshness measure of a foodstuff product from which the muscle sample were extracted.
- The step of correlating comprises comparing the determined $C_{Hx}$ to previously determined $C_{Hx}$'s in diluted supernatant prepared from similar foodstuff products with a known K-value and/or QIM to estimate a K-value and/or QIM for the foodstuff product from which the muscle sample were extracted.
- The correlation is based on predefined parameters of the process, such as volumes, concentrations and types of additives, on predefined voltammetric settings, and on correlation data characteristic for the species of a host animal of the muscle sample.

For the sake of biochemical analysis, it may be preferred that the process further comprises the step of determining a hypoxanthine content in the muscle sample from determined $C_{Hx}$ in the diluted supernatant. This may be performed by calculating backwards through the sample preparation process or by correlating the determined $C_{Hx}$ with measurements on similar muscle samples with hypoxanthine content also determined by other methods. This may provide the advantage of enabling calculation of contents of other ATP metabolites in the muscle sample.

The invention also relates to devices and kits enabling the application of the process according to the first aspect.

Thus, in a second aspect, the invention provides a device for estimating a freshness measure of a target product comprising perishable fish, poultry or meat, the device comprising:

a voltammeter capable of performing adsorptive stripping voltammetry; and

a data analysis unit comprising

- means for reading voltammograms recorded by the voltammeter on a target solution prepared from a muscle sample from the target product and standard hypoxanthine solutions, and calculating a hypoxanthine concentration ($C_{Hx}$) in the target solution;
- storage means holding a set of $C_{Hx}$'s being previously calculated from voltammograms recorded by adsorptive stripping voltammetry on solutions similar to the target solution prepared from a set of muscle samples from products similar to the target product with known freshness measures, and the known freshness measures;
- means for comparing the calculated $C_{Hx}$ of the target solution with the set of previously calculated $C_{Hx}$'s to estimate a freshness measure of the target product.

Typical fish species are consumer products such as salmon, trout, cod, carp, flounder etc. Solutions similar to the target solution are solutions prepared from meat samples in at least the substantially same way the, i.e. preferably using the sample preparation process applied in the process according to the first aspect. Similarly, products similar to the target product are at least substantially the same product based on the same type and species of animal, and processed in the same way if applicable. The person skilled in the art will understand that the product used in the data set acts as references, and that the target product should therefore not differ significantly in order for the correlation to yield valuable results.

It may be preferred that a working electrode of the voltammeter is a glassy carbon electrode with an electrolytic deposited mercury layer. This working electrode may provide the advantages of showing a better specificity against hypoxanthine, and thereby provide more precise results.

In a third aspect, the invention provides a kit for preparing a muscle sample for voltammetric measurement of hypoxanthine level, comprising

- One phial containing perchloric acid or trichlor acetic acid with a concentration in the range [0.2 - 1M].
- One phial containing potassium hydroxide or sodium hydroxide with a concentration in the range [0.5 - 2M].
- Two phials containing a buffer solution with a pH in the range [6 - 6.5].
- Two phials containing standard hypoxanthine solution, or two sets comprising crystalline hypoxanthine and buffer for mixture.

The set of instructions preferably comprise the sample preparation steps of the process according to the first aspect.

As can be understood from the previous, the determination of a freshness measure based on the voltammetric determined $C_{Hx}$ relies on a data set with previously recorded concentrations. Hence, in a fourth aspect, the invention provides a process for forming a data set to be used in estimating freshness measures of products comprising perishable fish, poultry or meat, the process comprising:

- providing a set of products of the same type;
- storing the set of products under controlled conditions, and selecting a product for analysis at predetermined intervals;
- for each selected product, determining a hypoxanthine concentration ($C_{Hx}$) by performing at least the following:

  ■ adding at least one acid to the sample to form a mixture having a pH<2, and homogenizing the resulting mixture to at least partly dissolve the sample;
  ■ adding a base to the homogenized mixture to precipitate proteins and thereby produce supernatant comprising ATP metabolites from the muscle sample;
  ■ diluting the supernatant with an buffer; and
  ■ subjecting the diluted supernatant to adsorptive stripping voltammetry to determine a $C_{Hx}$ in the diluted supernatant in comparison with a standard hypoxanthine solution;

- for each selected product, determining a freshness measure using a sensory and/or a non-sensory method; and
- forming a data set by storing pairs of corresponding freshness measures and determined $C_{Hx}$ s for each selected product.

  That the set of products are of the same type means that they are based on e.g. the same species etc. Since the set of products are used to form a reference set, using significantly different products does not make sense. Also, the muscle samples are preferably extracted from similar parts of the animal in order for comparison to be valuable. This implies that the time passed since the animal were killed is preferably similar for all products or should at least be controlled and taken into account when selecting products at the predetermined intervals. The predetermined intervals depends to some degree on the type of animal from which the product origins and on the storage method (at room temperature, at refrigerator temp (+5°), on ice, deep-frozen). Generally, by time on storage is meant time on ice or in refrigeration. For fish and, which is spoiled relatively fast, measurable spoilage may occur on a day-to-day basis, and the predetermined interval is preferably one or two days in order to generate a data set from which precise interpolation can be made. For poultry at un-frozen storage, the predetermined interval is preferably one or few days. For meat to become tender, longer storage times at temperatures above freezing are often required, and the predetermined interval may be several days.

The basic principle of the present invention is that $C_{Hx}$ can be directly determined by voltammetric measurements on a biological sample prepared from muscle tissue. Such determination further provides estimating a freshness measure for the product from which the muscle tissue is extracted, by correlating the determined $C_{Hx}$ to previously determined $C_{Hx}$'s for samples extracted from products with known freshness measures.

**Brief description of the figures**

Figure 1 shows the degradation of ATP in muscle after death.

Figure 2 illustrates an embodiment of a device for determining a freshness measure in accordance with an aspect of the invention, showing a potentiostat, a computer containing the application and calculation software, and a voltammetric cell with a three electrode configuration.

Figure 3 shows where samples from fish should be taken.

Figure 4 shows a correlation between K-value and $C_{Hx}$ as determined by voltammetry.

Figure 5 shows the correlation between QIM score and $C_{Hx}$ as determined by voltammetry.

Figure 6 shows the correlation between $C_{Hx}$ as determined by HPLC and $C_{Hx}$ as determined by voltammetry.

Figure 7 shows a voltammogram of a salmon sample extract with 2x standard addition.

Figure 8 shows the standard addition data corresponding to the voltammograms of Figure 7.

**Detailed description of the invention**

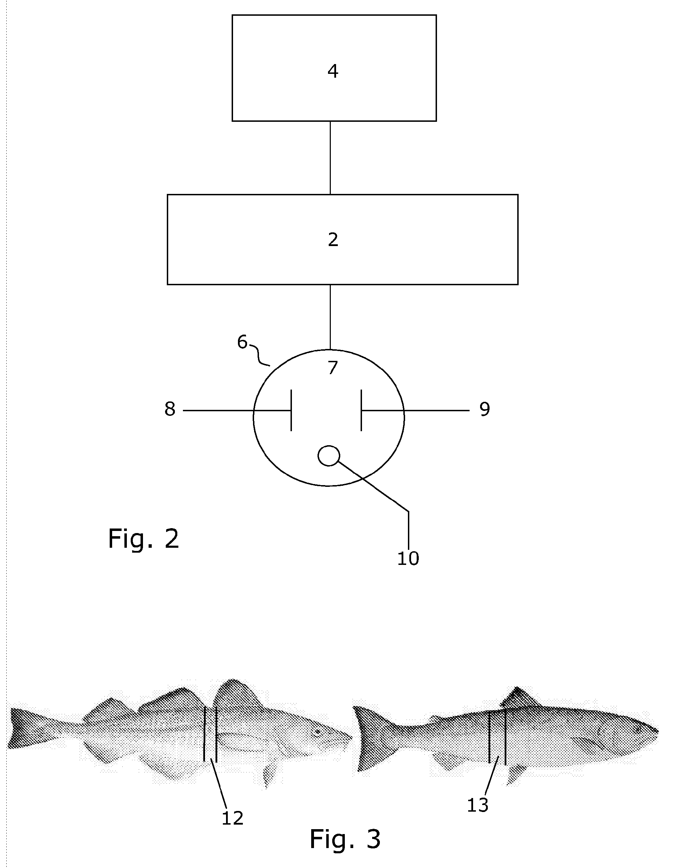

The present invention can apply commercially available voltammetry equipment or modified equipment specially adapted for the process according to the invention.
The basic components of a device for estimating a freshness measures according to the invention are illustrated in Figure 2 in the form of a system for performing voltammetry comprising; a voltammeter comprising a potentiostat 2 and an electrochemical cell 6, such as a voltammetric or polarographic cell, the voltammeter being in communication with a data analysis unit 4 such as a computer.
The polarographic cell 6 comprises a Teflon® coated or a glass sample container 7 in which are inserted a tube for bubbling of nitrogen and three electrodes:

a working (or indicator) electrode 8: a capillary connected with a mercury container (mercury electrode) or a solid ring disk electrode made of glassy carbon with a thin film of mercury.
an auxiliary (or counter) electrode 9: a platinum electrode or a carbon rood electrode.
a reference electrode 10: an Ag/AgCl, saturated KCl electrode.

The common characteristic of all voltammetric techniques is that they involve the application of a potential (E) to an electrode and the monitoring of the resulting current (i) flowing through the electrochemical cell. The applied potential is varied or the current is monitored over a period of time (t), and the resulting current is a function of E and t. The applied potential forces a change in the concentration of an electro active species at the electrode surface by electrochemically reducing or oxidizing it.
The task of applying a known potential and monitoring the current falls to the potentiostat. The potentiostat is configured to impose the desired potential scanning between working electrode 8 and reference electrode 10, while the current flowing in the circuit is recorded between working electrode 8 and auxiliary electrode 9.
The most widely used potentiostats today are assembled from discrete integrated-circuit operational amplifiers and other digital modules. In many cases, especially in the larger instruments, the potentiostat package also includes electrometer circuits, A/D and D/A converters, and dedicated microprocessors with memory. Hence, the potentiostat 2 and the computer 4 can be bundled into one package, whereas in other systems the computer and A/D and D/A converters and microcontroller are separate, and the potentiostat can operate independently.
The data analysis unit 4 includes or has access to a memory with a data set comprising $C_{Hx}$'s being previously calculated from voltammograms recorded on solutions prepared from meat products with known freshness measures (QIM, K-value etc.), and the corresponding known freshness measures. This data set can be used as a look-up table to estimate a freshness measure when knowing $C_{Hx}$. The data analysis unit 4 further holds and executes software comprising applications for performing the following functions:

- Sending the scanning parameters to the potentiostat.

- Checking if the execution of the scanning is correct or not.
- Receiving and storing the output data (current and potential), hereunder reading voltammograms recorded on a target solution prepared from a muscle sample from a meat product and standard hypoxanthine solutions.
- Calculating $C_{Hx}$ in the target solution
- Correlating the calculated $C_{Hx}$ with previously recorded values in the data set to determine a freshness measure of the meat product.

As will be described in relation to the examples later, the software on the data analysis unit can include the program package Metrodata from Metrohm Ltd (CH-9101 Herisau, Switzerland).

The size, power, sophistication, and price of voltammeters vary from large research-grade instruments (20 to 30 kg with a $\pm$10-volt potential and 1 A to 100 nA current ranges) to simple battery-powered units (3 to 1 kg with a $\pm$2.5-volt potential and 6 mA to 50 pA current ranges).

Many different forms of voltammetry exists, each of which provide specific chemical, electrochemical and physical information, such as quantitative determinations. The uniqueness of each rests on subtle differences in the manner and timing in which the potential is applied and the current measured. For quantitative determination, the interpretation of the recorded current trace is highly dependent upon the form of voltammetry and the applied electrode, in some cases, the concentration of the analyte is proportional to a plateau current, in others, the concentration is proportional to a peak height.

The following shortly presents the voltammetry forms preferred in the various aspects of the process according to the present invention, namely differential pulse adsorptive stripping voltammetry and linear sweep adsorptive stripping voltammetry. The details of applying these techniques to pure samples are well known to the skilled person.

In differential pulse voltammetry, the applied potential is a series of pulses (typically small amplitude of 10 to 100 mV) superimposed on a slowly changing base potential. The current is measured at two points for each pulse, the first point just before the application of the pulse and the second at the end of the pulse. The difference between current measurements at these points for each pulse is determined and plotted against the base potential.

Linear sweep voltammetry is the classic, simplest voltammetric measurement, but with limited sensitivity. In linear sweep voltammetry the direct voltage applied to the working electrode 8 is continuously changed and the resultant current which flows is measured. Linear sweep voltammetry is also called sample direct current voltammetry.

Adsorptive stripping voltammetry (AdSV) is a pre-concentration technique with a very high sensitivity. The hypoxanthine molecules are accumulated on the electrode surface of a mercury electrode. The adsorbed molecules can be estimated by stripping them from the electrode surface using linear sweep, staircase or differential pulse voltammetry. AdSV can be done on mercury electrodes and on mercury thin film electrodes (MTFE)

A device for determining the freshness of fish according to the invention need only measure a well defined current trace using a well defined form of voltammetry. Hence, the overall flexibility and the requirements to dynamical ranges in the applied potential and current sampling of the voltammeter can be drastically reduced without loss.

A brief description of the steps of the processes for determining a hypoxanthine content or for forming a data set to be used in estimating freshness according to the invention performed by the device shown in Figure 2. Preceding these steps lie the steps of extracting a meat sample and preparing the diluted supernatant therefrom, which are typically carried out manually or by a robot. These preceding steps will be described in greater detail later in relation to the examples and recipe.

The data analysis unit 4 runs the voltammetry sequence on the diluted supernatant in the sample vessel 7; receives the recorded current traces from the voltammeter; and calculates $C_{Hx}$ in the diluted supernatant. Software for performing this task is well known from commercial voltammetry systems and may be incorporated into the data analysis unit.

To be able to correlate the determined $C_{Hx}$ to a freshness measure, a data set holding previously recorded $C_{Hx}$ for similar products with known freshness measures has been prepared and is held either by the data analysis unit or by an accessible remote unit (e.g. a network server). The data set may be a table, a graph or an equation derived by applying a regression model to the previously recorded concentrations and freshness measures. By looking up the $C_{Hx}$ in the data set, a freshness measure for the product from which the muscle sample was extracted can be estimated, possibly by interpolation or extrapolation.

The result of the analysis, i.e. a freshness measure for the product from which the muscle sample was extracted, is communicated by an output device, e.g. a display, a printer or a network connection providing the freshness measure to another system such as a in a fish market or a slaughterhouse.

## Examples

In the following, the process for forming the previously recorded data set is described. This process also illustrates steps in the process for voltammetric determination of $C_{Hx}$ for biological samples according to the invention and the steps of correlating, comparing and estimating in accordance with a preferred embodiment of the invention.

<u>Muscle samples and storage regime</u>

Fresh (1 day after slaughtering) raised salmon and cod is stored at 4°C in triplets on ice. Analyses are performed at 1-3 days intervals for 15-18 days, and consist of determining freshness measures and $C_{Hx}$ for fish of similar freshness. The methods in use are QIM, the HPLC method for determining a K-value, and the voltammetric determination of $C_{Hx}$ according to the invention.

The QIM method

The technique is based on selecting a number of quality attributes characteristic for a particular species and allocating scores to each attribute depending on the state of freshness or quality of the selected food item. The scores are assigned in whole numbers ranging from 0, for fresh, to 3 dependent on the advancement of the decay. A quality Index (QI) is obtained by summarising the scores for all categories. The most commonly used attributes for fish are the appearance of eyes, skin and gills together with odour and texture. As an example the QIM scheme developed for farmed salmon is reproduced in Table 2 from Sveinsdottir et al., Quality Index Method (QIM) scheme developed for farmed Atlantic salmon (Salmo salar), Food Qual. Pref. 14 (2003), 237-245.

Table 2. The QIM scheme for farmed salmon (Sveisdottir et al. 2003)

| Quality parameters | Description | P | Quality parameters | Description | P |
|---|---|---|---|---|---|
| **Skin** | | | **Abdomen** | | |
| Colour/ | | | Blood in abdomen | Blood light red/not present | 0 |
| appearance | Pearl-shiny all over the skin The head is still pearl-shiny, but the rest | 0 | | Blood more brown | 1 |
| | less, perhaps yellow | 1 | Odour | Neutral | 0 |
| | | | | Corn | 1 |
| Mucus | Clear and not clotted | 0 | | Sour | 2 |
| | Milky and clotted | 1 | | Rotten/rotten kake | 3 |
| | Yellow and clotted | 2 | | | |
| Odour | Fresh seaweed, cucumber | 0 | **Gills**[b] Colour/ appearance | Red/dark brown | 0 |
| | Neutral to metal, dry grass, corn | 1 | | Light red/brown | 1 |
| | Sour | 2 | | Grey-brown, grey, green | 2 |
| | Rotten | 3 | | | |
| | | | Mucus | Transparent | 0 |
| **Eyes** | | | | Yellow, clotted | 1 |
| Pupils | Clear and black, mental shiny | 0 | | Brown | 2 |
| | Dark grey | 1 | | | |
| | Mat, grey | 2 | Odour | Fresh, seaweed | 0 |
| | | | | Metal | 1 |
| Form | Flat | 0 | | Sour | 2 |
| | Little sunken | 1 | | Rotten | 3 |
| | Sunken | 2 | | | |
| | | | **Texture** | | |
| | | | | Finger marks | |

(continued)

| | Texture | | |
|---|---|---|---|
| | Elasticity | disappears immediately | 0 |
| | | Finger leaves mark over 3 s | 1 |

Quality Index Total 0-22

[a]Turn the salmon and smell the skin on the other side.

[b] Examine the side that has not been cut through.

HPLC method for determining ATP metabolites and K-value

The following describes sample preparation for HPLC determination of ATP metabolite concentrations that can be used to calculate to a K-value.

Fish skin is removed and fish samples (doublets of approx. 0.6g), dissolved in perchloric acid (5 ml, 0.42M) and homogenized by an ultra turax (2 min, 9500 rpm).

All biological tissue and solutions are kept on ice between the manipulations are kept on ice between the manipulations. After precipitation with potassium hydroxide (1.5ml, 1.0M) the mixture is turned upside down and spun (12 000 rpm, 3 min). The supernatant is percolated through a syringe filter ($0.20\mu m$) and samples ($20\mu l$) are immediately injected in the HPLC.

The described process uses an isocratic HPLC-method to determine the K-value as described in Sellevold et al. High performance liquid chromatography: a Rapid Isocratic method for determination of creatine compounds and adenine nucleotides in myocardial tissue. J. Mol. Cell. Cardiol, 18 (1986), 517-527. The column is a Phenomenex reverse phase column (Synergi $4\mu$, Hydro RP 80A, 150x4.6,) and the mobile phase contains potassium dihydrogen phosphate (KH2PO4, 215mM), tetrabutylammonium hydrogen sulphate (TBAHS, 2.3mM and acetonitril 3.5%. The pH is adjusted to 6.25 with KOH. The chromatograms are performed at ambient temperature and the diode array detector is set to 206 and 260nm. The flow rate is maintained at 1.0 ml/min. External standards of hypoxanthine (0, 25, 50, 100, 200, 250 $\mu M$) are used to make a 6-point calibration curve to calculate the concentration of the nucleotide ATP metabolites.

The content of the various ATP metabolites are determined from the chromatograms and a K-value can be calculated using equation (1).

Voltammetric determination of $C_{Hx}$

The sample preparation for the voltammetric determination of $C_{Hx}$ applied in the present invention is described in the following.

A fish muscle sample is taken by slicing behind the first dorsal fin next to the head, as illustrated by segments 12 and 13 in Figure 3. Fish skin is removed and fish samples (doublets of approx. 0.6g), dissolved in perchloric acid (5 ml, 0.42M, pH<0.5) and homogenized by an ultra turax (2 min, 9500 rpm).

All biological tissue and solutions are kept on ice between the manipulations. After precipitation with potassium hydroxide (1.5ml, 1.0M), pH in the resulting solution is less than 1.0. The extract is turned upside down and put on ice and leaved to rest in 3 minutes. The supernatant is diluted 1:100 or 1:10 with phosphate buffer (pH 6.2 - 6.5), dependent on if the differential pulse adsorptive stripping voltammetry or the linear sweep voltammetry, respectively, is used, before percolating through a $0.20\mu m$ syringe filter (Chromacol 30-SF-02).

The supernatant holds six nucleotides (ATP and the first five decomposed nucleotides) and possibly some smaller water-soluble peptides. The presence of nucleotides were determined by performing a HPLC analysis of the fish sample supernatant prepared as described here, and detected at 260nm. It was also determined that when extracts are kept on ice, the decline in $C_{Hx}$ is negligible for at least 3-4 hours. Hypoxanthine is the only of these nucleotides which can be precisely determined by voltammetric analysis, without interference of ATP, ADP; AMP, IMP or inosin.

The voltammetric measurements on the diluted supernatant can be carried out using two different working electrodes. Both a pure mercury electrode (HMDE) and a mercury thin film electrode (MTFE) have been tested. All the voltammetric measurements were carried out using a 797 VA Computrace instrument from Metrohm (Metrohm Ltd, CH-9101 Herisau, Switzerland). The instrument was connected to a personal computer running a software package from Metrohm called Metrodata. All measurement and calculation were done with the use of this software.

*Measurements with a HMDE working electrode*

The multi-mode electrode from Metrohm was used as working electrode (dropping mercury electrode). The result was obtained using the differential pulse mode with pulse amplitude of -50 mV and with sample direct current voltammetry (Linear sweep voltammetry). Potentials were measured versus a silver /silver chloride / potassium chloride (3 mol/L) reference electrode, using a three-electrode system. The third electrode was a platinum wire.

Instrumental settings:

| Differential pulse mode | |
| --- | --- |
| Pulse amplitude | 50 mV |
| Deposition time | 30-60s |
| Deposition potential | 0.0 V |
| Start potential | 0.0 V |
| End potential | -0.4 V |
| Scan rate | -2 mV/s |
| Drop size | 4 |
| Stirrer speed | 2000 rpm |
| Initial purge time | 300 s |

| Sample direct current mode (linear sweep voltammetry) | |
| --- | --- |
| Cleaning potential | -1.2 V |
| Deposition time | 30-60s |
| Deposition potential | 0.0 V |
| Start potential | 0.0 V |
| End potential | -0.4 V |
| Scan rate | -1 mV/s |
| Drop size | 4 |
| Stirrer speed | 2000 rpm |
| Initial purge time | 300 s |

*Measurements with a MTFE working electrode*

The formation of mercury film was done by polishing the glassy carbon electrode with alumina slurry.
The mercury deposition was achieved by holding the electrode for 60 s at 1.20 V in an 0.001 M mercuric nitrate aqueous solution in 0.1 M HCl. Before each analysis, the MTFE was activated by holding at a potential of -1.5 V in an quiescent solution for 60 s.
The MTFE electrode was used as working electrode (dropping mercury electrode). The result was obtained using sample direct current voltammetry (Linear sweep voltammetry) and by differential pulse voltammetry. Potentials were measured versus a silver / silver chloride / potassium chloride (3 mol/L) reference electrode (Metrohm), using a three-electrode system. The third electrode was a carbon rood (Metrohm). The instrumental setting was as shown above.
The voltammetric cell was added 9.90 ml phosphate buffer and 100 $\mu$l diluted and percolated sample extract and analysed. The amount of hypoxanthine was determined by standard addition of 2 x 25 $\mu$l 0.002 mM or 0.01mM Hx standard for analysing by differential pulse adsorptive stripping voltammetry or by sampled direct current (linear sweep) voltammetry, respectively.

Results

Salmon samples were extracted and stored on ice for 9 days, and were scored in triplets by QIM on day 1, 5, 7, and 9. On the same days, $C_{Hx}$ in the same samples in doublets were determined by both HPLC and voltammetry as described in the above.
The results are analysed to determine whether:

1. $C_{Hx}$ determined by voltammetry correlates with K-value determined by HPLC.
2. $C_{Hx}$ determined by voltammetry correlates with the QIM score.

3. $C_{Hx}$ determined by voltammetry correlates with $C_{Hx}$ determined by HPLC.

Table 3. $C_{Hx}$ determined by voltammetry and HPLC in salmon samples stored on ice, and corresponding K-values and QIM scores.

| | $C_{Hx}$ [mg/g] | | | | K-value from HPLC | QIM | |
|---|---|---|---|---|---|---|---|
| Storage | Voltammetry | St. dev. | HPLC | St. dev. | [%] | Score | St. dev. |
| 1 day | 0,100 | 0,018 | 0,090 | 0,019 | 57,1 | 0,00 | 0,00 |
| 5 days | 0,157 | 0,012 | 0,134 | 0,023 | 72,5 | 7,00 | 2,00 |
| 7 days | 0,176 | 0,020 | 0,189 | 0,033 | 84,8 | 11,33 | 0,58 |
| 9 days | 0,195 | 0,018 | 0,210 | 0,015 | 87,9 | 13,67 | 2,31 |

Table 3 gives the determined $C_{Hx}$ values and QIM scores and their standard deviations for the doublets/triplets, as well as the K-values calculated from the ATP metabolites determined by HPLC.

The values in Table 3 reveal coherence between $C_{Hx}$'s determined by voltammetry and by HPLC. A t-test reveals no significant differences between the two methods (P<0.03) and the correlation is calculated to 0.984. The values in Table 3 also reveal coherence between the K-value and the $C_{Hx}$ determined by HPLC. The correlation is calculated to 0.950.

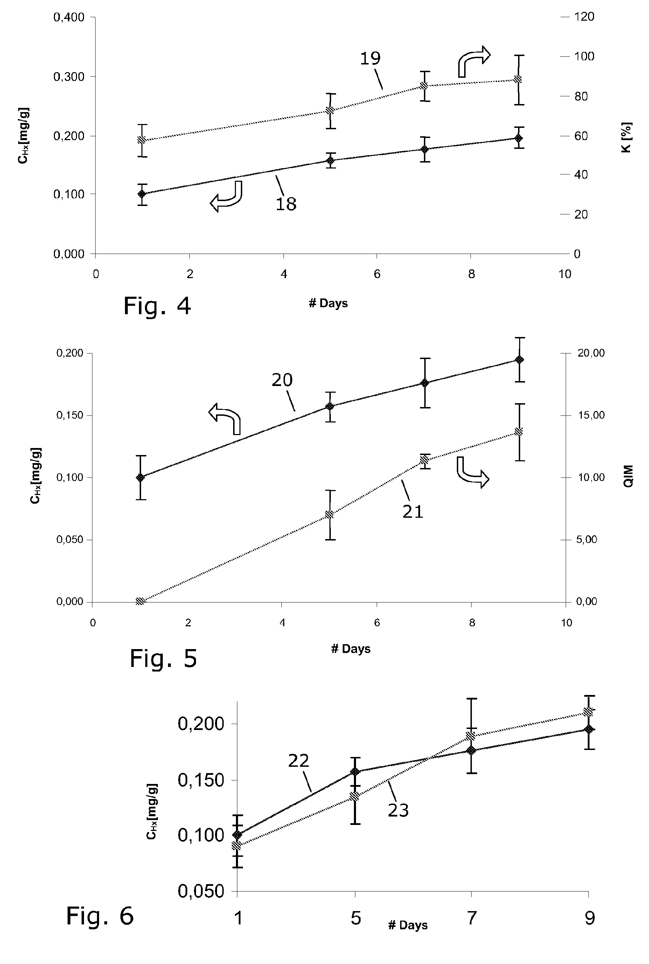

Figure 4 shows a graph comparing the $C_{Hx}$'s determined by voltammetry (curve 18) and the corresponding K-values determined by HPLC (curve 19); the correlation is calculated to be 0.984.

Figure 5 shows a graph comparing the $C_{Hx}$'s determined by voltammetry (curve 20) and the corresponding QIM scores (curve 21); the correlation is calculated to be 0.993.

Figure 6 shows a graph comparing the $C_{Hx}$'s determined by voltammetry (curve 20) and the corresponding $C_{Hx}$'s determined by HPLC (curve 21); the correlation is calculated to be 0.963.

As can be seen, the correlation between $C_{Hx}$'s determined by voltammetry and HPLC are almost the same as between $C_{Hx}$'s determined by voltammetry and K-value. This indicates that the inclusion of the other ATP metabolites from Equation (1) does not play an important role, and that the $C_{Hx}$'s determined by voltammetry alone can be used as a good freshness measure.

In the data presented in the above, it is the $C_{Hx}$ in the fish muscle which is correlated to the freshness measure. The analysed $C_{Hx}$ in the fish muscle depends on the exact preparation recipe, an example of which will be described in detail in the below. Hence, it may be of interest to calculate the Hypoxanthine content in the extracted muscle sample, e.g. in weight % or in mol/g. This Hypoxanthine content may be correlated with different freshness measures as in the above. This provides the advantage of allowing direct comparison between Hypoxanthine content in the meat product as determined by different methods.

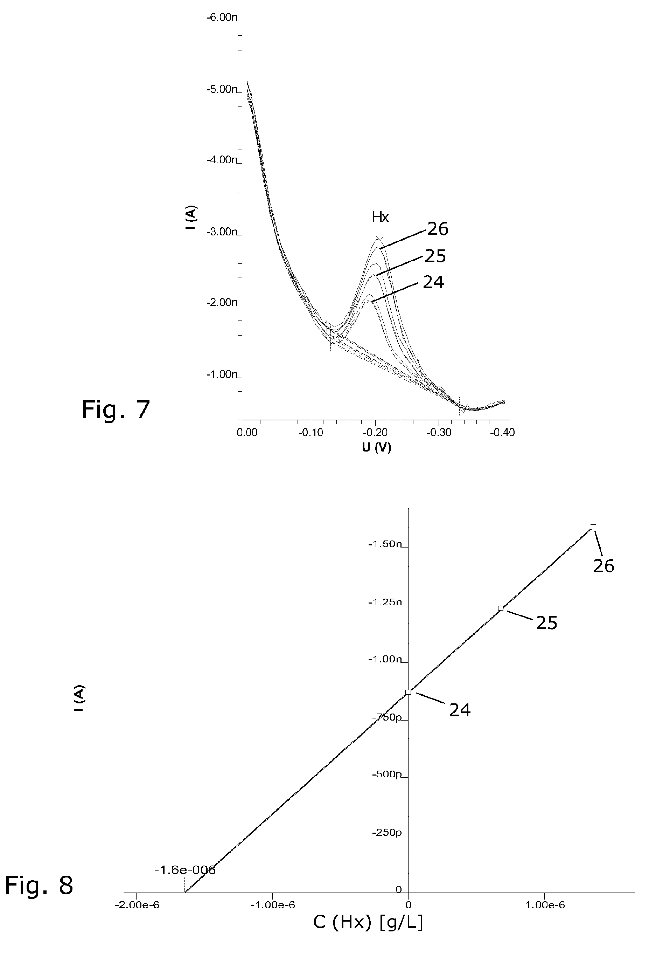

Figure 7 shows voltammograms of a salmon sample extract (9.90 ml phosphate buffer and 100 $\mu$l diluted and percolated sample extract) analysed in triplicate (24) and then added 25 $\mu$l 0.002 mM Hx standard solution and analysed in triplicate (25), and again added 25 $\mu$l 0.002 mM Hx standard solution and analysed in triplicate (26). The analysis was done with a Hg-electrode using differential pulse adsorptive stripping voltammetry. The salmon sample is extracted from an 8 days old salmon.

Figure 8 shows the corresponding standard addition data from figure 7. The peak current height, measured in pico and nano ampere, is plotted versus the standard addition concentration of hypoxanthine. The mean value of the triplicate peak height of the sample (24) and 2x standard addition of 25 $\mu$l 0.002 mM Hx standard solution (25 and 26) is plotted.

**Recipe and kit**

In the following, a general recipe for the process for determining $C_{Hx}$ in biological samples according to the invention is given. References to the above examples are used for putting the recipe into context.

Sample extraction

For fish species, a muscle sample is taken by slicing behind the first dorsal fin next to the head (Figure 3). For other marine or land animal the sample is collected from dense muscle without to much fat. Skin and surplus fat or other tissue is preferably removed to reduce the sample size (such surplus tissue will typically not affect the determination, but may be inconvenient in the following steps). Samples can be taken in doublets to increase the statistics. Typical recommended sample size for fish species is 0.6 - 1.0 g. The ATP degradation in extracted muscle samples is similar to that in muscles in the dead animal, but the increased exposure to light and air may speed up the degradation. Therefore, all biological tissue and solutions are kept on ice between manipulations, and extracted samples are preferably dissolved immediately after extraction.

Dissolving

After extraction, the muscle sample is dissolved in a strong acid, e.g. perchloric acid or trichlor acetic acid. Preferred concentrations of the strong acid are in the ranges [0.1 - 2M]; [0.1 - 1.5M]; [0.1 - 1M]; [0.3 - 2M]; [0.4 - 2M]; [0.3 - 1,5M]; [0.2 - 1M], such as in the range [0.3 - 1M], [0.3 - 0.8M], or [0.4 - 0.6M]. The applied volume of strong acid depends, among other things, on the concentration and the sample size. Preferably, the volume is in the range [2 - 10 ml], such as [4 - 6mL]. With the recommended sample size, 5 ml perchloric acid or trichlor acetic acid in the range [0.4 - 0.6M] is preferably used. The sample and acid is homogenized by a cell disrupter, such as by an Ultra Turax (2 min, 9500 rpm). The mixture obtained typically has a pH<1, but may be higher.

Precipitation

Proteins are precipitated with a strong hydroxide base raising the pH. Preferred concentrations of the strong base are in the ranges [0.5 - 3M]; [0.5 - 2.5M]; [1 - 3M]; [1 - 2.5M]; [0.5 - 2M]; [1 - 2M]; [0.5 - 1.5M]; preferably [1 - 1.5M]. The applied volume of strong base depends, among other things, on the base concentration and the sample size, and upon the strong acid used in the previous dissolving. Preferably, the volume is in the range [0.5 - 3ml], such as [1 - 2mL]. With the recommended sample size, 1.5mL potassium- or sodium hydroxide in the range [1.0-1.5M] is preferably used. The resulting mixture is still acidic (pH ~ 2), and is turned upside down and put on ice and leaved to rest.

Dilution

The supernatant is diluted so that the anticipated $C_{Hx}$ will lie in the linear regime of the voltammetric method. In the examples described in the above, typical dilution with the sample size and doses proposed herein are in the area 1:100 for the differential pulse adsorptive stripping voltammetry, and 1:10 or 1:20 for the sampled direct current linear sweep voltammetry. A phosphate buffer with a neutral pH (preferably in the range [6.00-6.50], such as [6.20-6.35]) is used in order to obtain a pH in an appropriate regime to the voltammetric method. The preferred buffer is a 0.1 M potassium phosphate - sodium hydroxide buffer. Such buffer can be prepared by mixing $KH_2PO_4$ (50 ml, 0.2M) and NaOH (8.6 ml, 0.2M) and dilute to 100 ml with water. The dilution typically takes place in two steps in order to avoid too large volumes, the above being the first step.

Percolation

The precipitate is removed before the supernatant transferred to the voltammetric cell. The supernatant is preferably percolated, typically through a syringe filter, simultaneously with extracting a sample after the first dilution step. In the examples described in the above, a 0.2 μm syringe filter (Chromacol 30-SF-02 (N), Chromacol LTD, Welwyn Garden City, UK) was used. In a preferred embodiment, the syringe filter is a protein filter, made of cellulose acetate is suitable for filtration of hydrophilic biological samples.

Voltammetry

The supernatant is added to the voltammetric cell, typically together with more phosphate buffer in a second dilution step (e.g. 9.90ml buffer 100μl supernatant), resulting in a mixture with a neutral pH, preferably in the range [6.00 - 6.50], preferably [6.20-6.35]. The preferred volume of the resulting mixture depends on the type of voltammeter cell used.
The $C_{Hx}$ is determined by adsorptive stripping voltammetry by standard addition of Hx standard solution. In the above examples, the Hx standard solutions were 2x25μl 0.002mM for differential pulse adsorptive stripping voltammetry, and 2x25μl 0.01mM for sampled direct current linear sweep voltammetry. The applied volume of Hx standard solution depends, among other things, on the sample size, anticipated Hx content, and total volume in the voltammetric cell. Typically, two equal volumes in the range 10-100μL are used. Applicable working electrodes are Hg-electrode, MTFE, and possibly amalgam electrodes.

**Kit**

In the following, an example of a kit for preparing a muscle sample for voltammetric measurement of hypoxanthine is described. The volumes and concentrations of the example are chosen in accordance with the sample size and meat product described in the above, hence, other volumes and/or concentrations disclosed previously and performing the same functions may be used.

The kit:

- One phial containing 2-10 ml, perchloric acid or trichlor acetic acid with a concentration in the range [0.2 - 1M].
- One phial containing 0.5 - 3 ml, potassium hydroxide or sodium hydroxide with a concentration in the range [0.5 - 2M].
- Two phials containing a buffer solution with a pH in the range [6 - 6.5].
- Two phials containing 10-100$\mu$L standard hypoxanthine solution, or two sets comprising crystalline hypoxanthine and 10-100$\mu$L buffer for mixture.

The kit can also comprise a syringe filter: (0.20$\mu$m) to be used in the percolation, as these are typically throwaway filters.

The kit can also comprise a set of instructions on how to prepare the diluted supernatant according to the recipe described in the above.

The kit can also comprise correlation tables or graphs between the $C_{Hx}$ readout from the voltammeter and preferred freshness measures. This may be provided electronically so that the data analysis unit connected to the voltammeter can make the correlation automatically.

**Claims**

**1.** Process for determining a hypoxanthine content in a muscle sample comprising:

adding at least one acid to a provided muscle sample to form a mixture having a pH<2, and homogenizing the resulting mixture to at least partly dissolve the sample;
adding a base to the homogenized mixture to precipitate proteins and produce a supernatant comprising ATP metabolites from the muscle sample;
diluting the supernatant with an buffer; and
subjecting the diluted supernatant to adsorptive stripping voltammetry to determine a hypoxanthine concentration ($C_{Hx}$) in the diluted supernatant in comparison with a standard hypoxanthine solution.

**2.** The process according to claim 1, wherein no further separation of the ATP metabolites is performed prior to the voltammetry.

**3.** The process according to any of the preceding claims, wherein the acid is selected from the group consisting of perchloric acid and trichloracetic acid.

**4.** The process according to any of the preceding claims, wherein the base is selected from the group consisting of potassium hydroxide and sodium hydroxide.

**5.** The process according to any of the preceding claims, further comprising the step of correlating the determined $C_{Hx}$ to a freshness measure of a foodstuff product from which the muscle sample were extracted.

**6.** The process according to claim 5, wherein the step of correlating comprises comparing the determined $C_{Hx}$ to previously determined $C_{Hx}$'s in diluted supernatant prepared from similar foodstuff products by adsorptive stripping voltammetry with a known freshness measure to estimate a freshness measure for the foodstuff product from which the muscle sample were extracted.

**7.** The process according to any of the preceding claims, wherein the freshness measure is selected from the group consisting of number of days on storage, K-value, QIM score and RT-freshmeter readout.

**8.** The process according to any of the preceding claims, further comprising step of determining a hypoxanthine content in the muscle sample from determined $C_{Hx}$ in the diluted supernatant.

**9.** The process according to any of the preceding claims, wherein diluting the supernatant with a buffer results in a diluted supernatant with a Ph in the range 6.00-6.50, preferably in the range 6.20-6.35.

**10.** The process according to any of the preceding claims, wherein the adsorptive stripping voltammetry used to determine $C_{Hx}$ is differential pulse adsorptive stripping voltammetry or sampled direct current linear sweep voltammetry.

**11.** Kit for preparing a muscle sample for voltammetric measurement of hypoxanthine level, comprising

- One phial containing perchloric acid or trichlor acetic acid with a concentration in the range [0.2 - 1M].
- One phial containing potassium hydroxide or sodium hydroxide with a concentration in the range [0.5 - 2M].
- Two phials containing a buffer solution with a pH in the range [6 - 6.5].
- Two phials containing standard hypoxanthine solution, or two sets comprising crystalline hypoxanthine and buffer for mixture.

**12.** The kit according to claim 11, further comprising a 0.20$\mu$m syringe filter.

**13.** The kit according to claim 11, further comprising a set of instructions on how to prepare the diluted supernatant according to the process of claim 1.

**14.** The kit according to claim 11, further comprising a correlation data between a $C_{Hx}$ determined by adsorptive stripping voltammetry and a freshness measure.

**15.** A device for estimating a freshness measures of a target product comprising perishable fish, poultry or meat, the device comprising:

a voltammeter capable of performing adsorptive stripping voltammetry;
a data analysis unit comprising

- means for reading voltammograms recorded by the voltammeter on a target solution prepared from a muscle sample from the target product and standard hypoxanthine solutions, and calculating a hypoxanthine concentration ($C_{Hx}$) in the target solution;
- storage means holding a set of $C_{Hx}$'s being previously calculated from voltammograms recorded by adsorptive stripping voltammetry on solutions similar to the target solution prepared from a set of muscle samples from products similar to the target product with known freshness measures, and the known freshness measures;
- means for comparing the calculated $C_{Hx}$ of the target solution with the set of previously calculated $C_{Hx}$'s to estimate a freshness measure of the target product.

**16.** The device according to claim 15, wherein a working electrode of the voltammeter is a glassy carbon electrode with an electrolytic deposited mercury layer.

**17.** A process for forming a data set to be used in estimating freshness measures of products comprising perishable fish, poultry or meat, the process comprising:

providing a set of products of the same type;
storing the set of products under controlled conditions, and selecting a product for analysis at predetermined intervals;
for each selected product, determining a hypoxanthine concentration ($C_{Hx}$) by performing at least the following:

- adding at least one acid to the sample to form a mixture having a pH<2, and homogenizing the resulting mixture to at least partly dissolve the sample;
- adding a base to the homogenized mixture to precipitate proteins and thereby produce supernatant comprising ATP metabolites from the muscle sample;
- diluting the supernatant with an buffer; and
- subjecting the diluted supernatant to adsorptive stripping voltammetry to determine a $C_{Hx}$ in the diluted supernatant in comparison with a standard hypoxanthine solution;

for each selected product, determining a freshness measure using a sensory and/or a non-sensory method; and forming a data set by storing pairs of corresponding freshness measures and determined $C_{Hx}$ s for each selected product.

**21.** The process according to claim 20, wherein the sensory and/or a non-sensory method for determining a freshness measure comprises at least one or more methods selected from the group consisting of: number of days on storage, K-value determined by HPLC, QIM score, RT-freshmeter readout.

Fig. 1

Fig. 2

Fig. 3

Fig. 4

Fig. 5

Fig. 6

Fig. 7

Fig. 8

**European Patent Office**

**EUROPEAN SEARCH REPORT**

Application Number

EP 06 12 3613

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,Y | JYH-MYNG ZEN , YU-YEN LAI: "Electrocatalytic Oxidation of Hypoxanthine on a Nafion/Lead-Ruthenium Oxide Pyrochlore Modified Electrode" ELECTROANALYSIS, vol. 12, no. 4, 1999, pages 280-286, XP002430829 * the whole document * | 1 | INV. G01N33/12 G01N27/403 |
| D,Y | IBRAHIM M.S ETAL: "Adsorptive stripping voltammetric behaviour of hypoxanthine" ANALYTICA CHIMICA ACTA, vol. 328, 1996, pages 47-52, XP002430830 * the whole document * | 1 | |
| D,A | OLIVEIRA-BRETT A.M, FARACE G. ET AL: "Voltammetric and impedance studies of inosine-5'-monophosphate and hypoxanthine" BIOELECTROCHEMISTRY, vol. 59, 2002, pages 49-56, XP002430831 * the whole document * | 1,15,17 | |
| D,A | US 5 288 613 A (LUONG JOHN H T [CA] ET AL) 22 February 1994 (1994-02-22) * the whole document * | 1,15,17 | TECHNICAL FIELDS SEARCHED (IPC) G01N |
| D,A | EP 0 125 923 A2 (ZAIDAN HOJIN SHOKUHIN SANGYO [JP]; ORIENTAL DENKI KK [JP]) 21 November 1984 (1984-11-21) * the whole document * | 1,15,17 | |

-/--

The present search report has been drawn up for all claims

| Place of search | Date of completion of the search | Examiner |
|---|---|---|
| The Hague | 24 April 2007 | Joyce, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office** — **EUROPEAN SEARCH REPORT**

Application Number

EP 06 12 3613

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZEN J-M ET AL: "Multianalyte sensor for the simultaneous determination of hypoxanthine, xanthine and uric acid based on a preanodized nontronite-coated screen-printed electrode" SENSORS AND ACTUATORS B, ELSEVIER SEQUOIA S.A., LAUSANNE, CH, vol. 84, no. 2-3, 15 May 2002 (2002-05-15), pages 237-244, XP004360395 ISSN: 0925-4005 * the whole document * | 1,15,17 | |

-----

TECHNICAL FIELDS
SEARCHED    (IPC)

The present search report has been drawn up for all claims

| Place of search | Date of completion of the search | Examiner |
|---|---|---|
| The Hague | 24 April 2007 | Joyce, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 921 447 A1**

## ANNEX TO THE EUROPEAN SEARCH REPORT
## ON EUROPEAN PATENT APPLICATION NO.

EP 06 12 3613

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2007

| Patent document cited in search report | | Publication date | Patent family member(s) | | Publication date |
|---|---|---|---|---|---|
| US 5288613 | A | 22-02-1994 | NONE | | |
| EP 0125923 | A2 | 21-11-1984 | CA | 1219796 A1 | 31-03-1987 |
| | | | DE | 3480731 D1 | 18-01-1990 |
| | | | JP | 1721278 C | 24-12-1992 |
| | | | JP | 4003960 B | 24-01-1992 |
| | | | JP | 59232097 A | 26-12-1984 |
| | | | US | 5024816 A | 18-06-1991 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5024816 A
- US 4105800 A
- US 5288613 A
- EP 0137677 A

- DE 3909530
- JP 62288561 B
- JP 63015151 B

**Non-patent literature cited in the description**

- evaluation of fish freshness. AIR3CT94 2283. 396
- **SAITO et al.** A new method for estimating the freshness in fish. *Bull. Jpn. Soc. Sci. Fish.,* 1959, vol. 24, 749-753
- **OLIVEIRA-BRETT et al.** Voltammetric and impedance studies of inosine-5'-monophosphate and hypoxanthine. *Bioelectrochemistry,* 2003, vol. 59, 49-56
- **IBRAHIM et al.** Adsorptive stripping voltammetric behaviour of hypoxanthine. *Analytica Chimica Acta,* 1996, vol. 328, 47-52

- **JYN-MYNG ZEN et al.** Electrocatalytic oxidation of hypoxanthine on a nafion/Lead-Ruthenium oxide pyrochlore modified electrode. *Electroanalysis,* 2000, vol. 12, 280-286
- **SVEINSDOTTIR et al.** Quality Index Method (QIM) scheme developed for farmed Atlantic salmon (Salmo salar). *Food Qual. Pref.,* 2003, vol. 14, 237-245
- **SELLEVOLD et al.** High performance liquid chromatography: a Rapid Isocratic method for determination of creatine compounds and adenine nucleotides in myocardial tissue. *J. Mol. Cell. Cardiol,* 1986, vol. 18, 517-527